(19) Europäisches Patentamt
European Patent Office
Office européen des brevets

(11) **EP 2 432 402 B1**

(12) **EUROPÄISCHE PATENTSCHRIFT**

(45) Veröffentlichungstag und Bekanntmachung des Hinweises auf die Patenterteilung:
**27.04.2016 Patentblatt 2016/17**

(21) Anmeldenummer: **10724126.7**

(22) Anmeldetag: **12.05.2010**

(51) Int Cl.:
**A61B 17/16** *(2006.01)*    **A61B 17/84** *(2006.01)*

(86) Internationale Anmeldenummer:
**PCT/IB2010/001089**

(87) Internationale Veröffentlichungsnummer:
**WO 2010/133933 (25.11.2010 Gazette 2010/47)**

(54) **Vorrichtung zum Einbringen eines gebogenen Nagels in einen Knochen**

Device for introducing a bent nail into a bone

Dispositif permettant l'introduction d'un clou courbe dans un os

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **18.05.2009 CH 777092009**

(43) Veröffentlichungstag der Anmeldung:
**28.03.2012 Patentblatt 2012/13**

(73) Patentinhaber: **Biedermann Technologies GmbH & Co. KG**
**78166 Donaueschingen (DE)**

(72) Erfinder: **KLAUE, Kaj**
**CH-6942 Savosa (CH)**

(74) Vertreter: **Lusuardi, Werther**
**Dr. Lusuardi AG**
**Kreuzbühlstrasse 8**
**8008 Zürich (CH)**

(56) Entgegenhaltungen:
WO-A1-2008/099176    DE-C- 133 162
DE-U1- 7 607 981    US-A- 1 630 239
US-A1- 2007 225 721    US-A1- 2008 140 078

EP 2 432 402 B1

**Beschreibung**

Die Erfindung bezieht sich auf eine Vorrichtung gemäss dem Oberbegriff des Patentanspruchs 1 und auf einen Kit umfassend eine Vorrichtung und mindestens ein als Knochennagel ausgebildetes Implantat für die Ferse gemäss dem Patentanspruch 7.

Es besteht schon längere Zeit ein Bedürfnis in kontrollierter Weise Bohrungen längs einer kreisbogenförmigen Bahn legen zu können. Speziell in der Knochenchirurgie stellt sich das Problem, wie man gebogene Implantate in kontrollierter Weise und ohne unnötigen Knochenverlust in den Knochen einbringen kann. Bis heute gab es kein Werkzeug mit welchem man exakt kreisbogenförmige Bohrungen legen konnte, welche die Einführung eines kreisbogenförmigen Implantats, insbesondere eine Nagels gestattet hätte. Nur eine exakt kreisbogenförmige Bohrung erlaubt die problemlose Einführung eines gleichgestalteten Implantats, da jede von der Kreisbogenform abweichende Gestalt der Bohrung das einzubringenden Implantat zum Verklemmen bringt.

Die Dokumente WO 2008/099176 A1, US 2008/0140078 A1, US 2007/0225721 A1, US 1,630,239 A und das Deutsche Gebrauchsmuster DE 76 07 981 offenbaren Vorrichtungen gemäß dem Oberbegriff des Anspruchs 1.

Die Erfindung löst die gestellte Aufgabe mit einer Vorrichtung, welche die Merkmale des Anspruchs 1 aufweist und mit einem Kit umfassend eine Vorrichtung und mindestens ein als Knochennagel ausgebildetes Implantat für die Ferse, welcher die Merkmale des Anspruchs aufweist.

Die durch die Erfindung erreichten Vorteile sind im Wesentlichen darin zu sehen, dass dank der erfindungsgemässen Vorrichtung eine exakt kreisbogenförmige Bohrung hergestellt werden kann.

Weitere vorteilhafte Ausgestaltungen der Erfindung können wie folgt kommentiert werden:

In einer speziellen Ausführungsform umfasst die Vorrichtung einen motorischen Antrieb für die flexible Welle. Dabei ist die flexible Welle an ihrem hinteren Ende mit der Antriebsmaschine verbunden, wobei die Einspannmittel der Antriebsmaschine axial fest am hinteren Ende des steifen Rohres angeordnet sind. Ferner ist der Aussendurchmesser der flexiblen Welle gegenüber dem Rohrinnendurchmesser derart dimensioniert, dass die flexible Welle nur ein geringes Spiel im Rohr aufweist. Durch dieses geringe Spiel und die Führung der flexiblen Welle auf ihrer gesamten Länge im Rohr ist gewährleistet, dass sich die schraubenlinienförmigen Drahtwindungen der flexiblen Welle durch das auf den Werkzeugkopf zu übertragende Drehmoment nicht aufweiten können und somit trotz der Biegeelastizität eine hohe Torsionssteifigkeit der flexiblen Welle gewährleistet ist. Die Vorrichtung kann mit einem Handgriff ausgestattet sein.

In einer anderen Ausführungsform umfasst die Vorrichtung eine Schubplatte, welche am hinteren Ende der flexiblen Welle fixiert ist und an das hintere Ende des Rohrs anlegbar ist. An der Schubplatte ist ein Antriebszapfen angeordnet, welcher in eine Antriebsmaschine einspannbar ist. Durch Ausüben einer Vorschubskraft auf die Antriebsmaschine kann somit das Rohr zusammen mit der flexiblen Welle vorwärts bewegt werden.

In einer weiteren Ausführungsform sind am vorderen Ende der flexiblen Welle Kupplungsmittel angebracht, mittels welcher der Werkzeugkopf an der flexiblen Welle lösbar ankuppelbar ist. Die flexible Welle ist mittels der Schubplatte axial fest am hinteren Ende des Rohres gelagert während der Werkzeugkopf axial am vorderen Ende des Rohres anliegt.

Die Montage der flexiblen Welle und des Werkzeugkopfes erfolgt gemäss den folgenden Schritten:

a) Die flexible Welle wird von hinten in das Rohr eingeführt und die Schubplatte, welche in einer weiteren Ausführungsform ringförmig als Schubring ausgebildet ist, wird entweder wie eine Mutter auf der flexiblen Welle nach vorne oder nach hinten gedreht, oder rasterweise durch ein Federsystem auf der flexiblen Welle von Rille zu Rille verschoben;

b) Die flexible Welle wird ein wenig nach vorne und aus der vorderen Rohröffnung geschoben und der Werkzeugkopf mit einem fixen Rastersystem eingehakt; und

c) Die Schubplatte oder der Schubring wird beispielsweise in der Ausführungsform mit Rastersystem auf der flexiblen Welle von hinten gegen das hintere Ende des Rohres geschoben, wodurch die flexible Welle gespannt wird, da der Werkzeugkopf am vorderen Ende des Rohres anliegt.

In wiederum einer anderen Ausführungsform ist der Werkzeugkopf allein durch die flexible Welle bewegbar.

In einer weiteren Ausführungsform beträgt der Krümmungsradius der Zentralachse des Rohres höchstens 240 mm, vorzugsweise höchstens 210 mm.

Gemäß der Erfindung beträgt der Krümmungsradius der Zentralachse des Rohres mindestens 130 mm, vorzugsweise mindestens 200 mm.

Gemäß der Erfindung weist die Krümmung für jeden infinitesimalen Abschnitt des steifen Rohres einen Krümmungsradius der Zentralachse auf, der mindestens 130 mm beträgt.

In einer weiteren Ausführungsform weist das Rohr einen Rohraussendurchmesser $d_a$ auf, welcher maximal 16 mm, vorzugsweise maximal 13 mm beträgt.

In nochmals einer weiteren Ausführungsform weist das Rohr einen Innendurchmesser $d_i$ im Bereich von 3 bis 7 mm auf.

In einer anderen Ausführungsform weist das Rohr eine Mantelstärke von mindestens 0,5 mm, vorzugsweise von mindestens 1,5 mm auf.

In wiederum einer anderen Ausführungsform weist das Rohr eine Rohrlänge von mindestens 5 cm und maximal 35 cm auf.

In einer weiteren Ausführungsform ist das Rohr aus einem rostfreien Stahl hergestellt.

In einer weiteren Ausführungsform ist das Rohr aus einem Material mit einem E-Modul von mindestens $150 \cdot 10^3$ N/mm² hergestellt und weist eine Biegesteifigkeit $E \cdot I$ von mindestens $1,3 \cdot 10^6$ N mm² auf.

Werkstoffeigenschaften aus Dubbel, Taschenbuch für den Maschinenbau, 21.Auflage, 2005, Springer-Verlag

Rostfreie Stähle: E-Modul $150 - 220 \cdot 10^3$ N/mm²

Für das Rohr ergeben sich folgende Werte für das axiale Flächenmoment 2. Grades und die Biegesteifigkeit:

Typischer Wert für die Biegesteifigkeit (Rohr mit Aussendurchmesser $d_a$ = 12 mm und Innendurchmesser $d_i$ = 7 mm):

axiales Flächenmoment 2. Grades [Rohr: $I = I_x = I_y = \pi (d_a^4 - d_i^4)/64$] für $d_a$ = 12 mm und $d_i$ = 7 mm wird $I_x$ = 900 mm⁴,
Biegesteifigkeit $E \cdot I = 135 - 198 \cdot 10^6$ N mm²,

Minimalwert für die Biegesteifigkeit (Rohr mit Innendurchmesser $d_i$ = 3 mm und Wandstärke 0,5 mm):

axiales Flächenmoment 2. Grades [Rohr: $I = I_x = I_y = \pi (d_a^4 - d_i^4)/64$] für $d_a$ = 4 mm und $d_i$ = 3 mm wird $I_x$ = 8,6 mm⁴,
Biegesteifigkeit $E \cdot I = 1,3 - 1,9 \cdot 10^6$ N mm²,

In einer anderen Ausführungsform weist der Werkzeugkopf einen Aussendurchmesser D auf, und das Verhältnis $d_a/D$ zwischen dem Rohraussendurchmesser $d_a$ und dem Aussendurchmesser D beträgt maximal 0,95, vorzugsweise maximal 0,92. Typischerweise beträgt das Verhältnis $d_a/D$ = 10/11.

In einer weiteren Ausführungsform umfasst die Vorrichtung zusätzlich eine am zu behandelnden Körperteil befestigbare Zielvorrichtung, welche eine kreisbogenförmige Führung aufweist zur beweglichen Aufnahme des steifen Rohres. Die Zielvorrichtung ist vorzugsweise als C-Bogen ausgebildet.

In einer weiteren Ausführungsform umfasst die Führung zwei oder mehr Zielringe. Die Zentren der Zielringe liegen auf einem Kreisbogen, dessen Radius dem Krümmungsradius der Zentralachse des Rohres entspricht und welcher einen Bogenwinkel zwischen mindestens 10 ° und höchstens 45° aufweist.

In einer anderen Ausführungsform umfasst die Zielvorrichtung ein an der Oberfläche eines Knochens anlegbares erstes Ende, ein zweites Ende und einen an der Oberfläche eines Knochens anlegbaren Fixationsschlitten. Der Fixationsschlitten ist am zweiten Ende der Zielvorrichtung bezüglich der bogenförmigen Zielvorrichtung in radialer Richtung verschiebbar gelagert, beispielsweise mittels einer im Wesentlichen in radialer Richtung verlaufenden Schwalbenschwanzführung.

In einer anderen Ausführungsform ist der Fixationsschlitten mit Kirschnerdrähten am Knochen befestigbar.

In einer weiteren Ausführungsform ist der Werkzeugkopf mit axialen Perforationen zur Abfuhr von Knochenspänen ausgestattet. Die Perforationen durchdringen den Werkzeugkopf in axialer Richtung und münden in den Hohlraum der als Hohlwelle ausgebildeten flexiblen Welle.

In einer speziellen Ausführungsform des Kits ist der Knochennagel aus nichtresorbierbarem Material, vorzugsweise aus rostfreiem Stahl oder Titan oder CrCo hergestellt.

Werkstoffeigenschaften aus Dubbel, Taschenbuch für den Maschinenbau, 21.Auflage, 2005, Springer-Verlag

a) rostfreie Stähle: E-Modul $150 - 220 \cdot 10^3$ N/mm²
b) Titan: E-Modul $100 - 105 \cdot 10^3$ N/mm²
c) Titanlegierungen: E-Modul $110 - 130 \cdot 10^3$ N/mm²

In einer Ausführungsform weist das als Knochennagel ausgebildete Implantat einen Aussendurchmesser $D_I$ von 9 bis 13 mm auf.

In einer weiteren Ausführungsform ist der vordere Teil des Knochennagels elastisch biegbar ausgebildet.

Bei einer anderen Ausführungsform ist der vordere Teil weniger steif ausgebildet als der hintere Teil. Im vorderen Teil des Knochennagels können auch Längsrillen vorgesehen sein, so dass der vordere Teil einen kleineren Querschnitt aufweist als der hintere Teil. Durch die Schwächung des Querschnitts ergibt sich eine Flexibilisierung des vorderen Teils gegenüber dem steifen hinteren Teil. Sowohl der vordere Teil als auch der hintere Teil des Knochennagels können 30 bis 70 % der Gesamtlänge des Knochennagels ausmachen.

In einer anderen Ausführungsform weist der Knochennagel mindestens im vorderen Teil einen Längsschlitz auf.

In wiederum einer anderen Ausführungsform weist der Knochennagel mindestens im vorderen Teil ein kleeblattförmiges Querschnittsprofil auf.

In einer anderen Ausführungsform ist der hintere Teil des Knochennagels steif ausgebildet ist.

In einer weiteren Ausführungsform umfasst der Knochennagel mindestens im vorderen Teil einen zur Längsachse parallelen Hohlraum. Der Knochennagel kann auch in der Spitzenhälfte rohrförmig ausgestaltet sein, vorzugsweise mit einer Wanddicke von 0,5 mm. Zudem kann der rohrförmig ausgebildete vordere Teil des Knochennagels einen Schlitz aufweisen.

Für den elastischen vorderen Teil des Implantates ergeben sich folgende Werte für das axiale Flächenmoment 2. Grades und die Biegesteifigkeit:

Minimales axiales Flächenmoment 2. Grades für Aussendurchmesser $D_l = 9$ mm und Wandstärke von 0,5 mm (Innendurchmesser $d_l = 8$ mm)

[Rohr: $I = I_x = I_y = \pi (D_l^4 - d_l^4)/64$]

Für $D_l = 9$ mm und $d_l = 8$ mm wird $I_x = 121$ mm$^4$

Maximales axiales Flächenmoment 2. Grades für Aussendurchmesser $D_l = 13$ mm und Wandstärke von 0,5 mm (Innendurchmesser $d_l = 12$ mm)

[Rohr: $I = I_x = I_y = \pi (D_l^4 - d_l^4)/64$]

Für $D_l = 13$ mm und $d_l = 12$ mm wird $I_x = 384$ mm$^4$

Werkstoffeigenschaften aus Dubbel, Taschenbuch für den Maschinenbau, 21. Auflage, 2005, Springer-Verlag

a) rostfreie Stähle: E-Modul 150 - 220 $10^3$ N/mm$^2$

Minimale Biegesteifigkeit $E \cdot I = 18,2 - 26,6 \cdot 10^6$ N mm$^2$,

Maximale Biegesteifigkeit $E \cdot I = 57,6 - 84,5 \cdot 10^6$ N mm$^2$,

b) Titan: E-Modul 100 - 105 $\cdot 10^3$ N/mm$^2$

Minimale Biegesteifigkeit $E \cdot I = 12,1 - 12,7 \ 10^6$ N mm$^2$,

Maximale Biegesteifigkeit $E \cdot I = 38,4 - 40,3 \cdot 10^6$ N mm$^2$,

c) Titanlegierungen: E-Modul 110 - 130 $\cdot 10^3$ N/mm$^2$

Minimale Biegesteifigkeit $E \cdot I = 13,3 - 15,7 \cdot 10^6$ N mm$^2$.

Maximale Biegesteifigkeit $E \cdot I = 42,2 - 49,9 \cdot 10^6$ N mm$^2$,

Die Biegesteifigkeit B berechnet sich gemäss Dubbel, Taschenbuch für den Maschinenbau, 21. Auflage, 2005, Springer-Verlag nach der Formel:

$$B = E \ x \ I \ , \ wobei$$

wobei I das axiale Flächenmoment 2. Grades in mm$^4$ und E das E-Modul in N / mm$^2$ ist.

Das axiale Flächenmoment 2. Grades I ist abhängig von der gewählten Biegeachse

In einer anderen Ausführungsform ist der vordere Teil des Knochennagels plastisch deformierbar.

In wiederum einer anderen Ausführungsform weist der vordere Teil des Knochennagels eine Biegesteifigkeit von mindestens $12 \cdot 10^6$ N mm$^2$, vorzugsweise mindestens $15 \cdot 10^6$ N mm$^2$ auf.

In einer weiteren Ausführungsform weist der vordere Teil des Knochennagels eine Biegesteifigkeit von höchstens $85 \cdot 10^6$ N mm$^2$ , vorzugsweise höchstens $60 \cdot 10^6$ N mm$^2$ auf. Der vordere Teil weist zweckmässigerweise höchstens 50 % der Steifigkeit des hinteren Teils auf.

In einer anderen Ausführungsform weist der Knochennagel eine Nagellänge $L_l$ von 12 - 50 cm auf.

In wiederum einer anderen Ausführungsform beträgt der Krümmungsradius des Knochennagels höchstens 240 mm, vorzugsweise höchstens 210 mm.

In wiederum einer anderen Ausführungsform ist das Verhältnis $L_l/D_l$ zwischen der Nagellänge $L_l$ und dem Durchmesser $D_l$ kleiner als 16, vorzugsweise kleiner als 14.

In einer weiteren Ausführungsform weist das Implantat mindestens eine Querbohrung zur Aufnahme einer Verriegelungsschraube auf, wobei die Längsachse der Querbohrung vorzugsweise in der Krümmungsebene des Knochennagels liegt.

In einer anderen Ausführungsform beträgt der Krümmungsradius des Knochennagels mindestens 130 mm, vorzugsweise mindestens 200 mm.

In einer anderen Ausführungsform umfasst der Knochennagel zwischen dem vorderen Teil und dem hinteren Teil einen intermediären Teil, dessen Steifigkeit gegen den hinteren Teil zunimmt.

Eine spezielle Verwendung der Vorrichtung ist ihr Einsatz für die tibio-calcaneare Arthrodese.

Die Erfindung und Weiterbildungen der Erfindung werden im Folgenden anhand der teilweise schematischen Darstellungen mehrerer Ausführungsbeispiele noch näher erläutert.

Es zeigen:

Fig. 1 eine perspektivische Ansicht einer Ausführungsform der erfindungsgemässen Vorrichtung;

Fig. 2 eine schematische Seitenansicht der erfindungsgemässen Vorrichtung nach Fig. 1;

Fig. 3 einen Ausschnitt mit dem Werkzeugkopf der erfindungsgemässen Vorrichtung nach Fig. 1;

Fig. 4 eine schematische Seitenansicht der erfindungsgemässen Vorrichtung nach Fig. 1 mit einer teilweise fertiggestellten Bohrung im Knochen;

Fig. 5 eine Ausführungsform eines teilweise in die Knochen eingeführten erfindungsgemässen Knochennagels;

Fig. 6 eine schematische Ansicht des erfindungsgemässen Knochennagels nach Fig. 2 in seiner Ausgestaltung als Implantat;

Fig. 7 eine schematische Ansicht von antero-lateral einer weiteren Ausführungsform des in den Knochen implantierten erfindungsgemässen Knochennagels;

Fig. 8 einen Querschnitt bei der Linie X - Y in Fig. 7;

Fig. 9 einen Querschnitt bei der Linie Z - Y in Fig. 7;

Fig. 10 einen Querschnitt bei der Linie W - Y in Fig.

7; und

Fig. 11a - 11k schematische Darstellungen einer Ausführungsform des erfindungsgemässen Verfahrens.

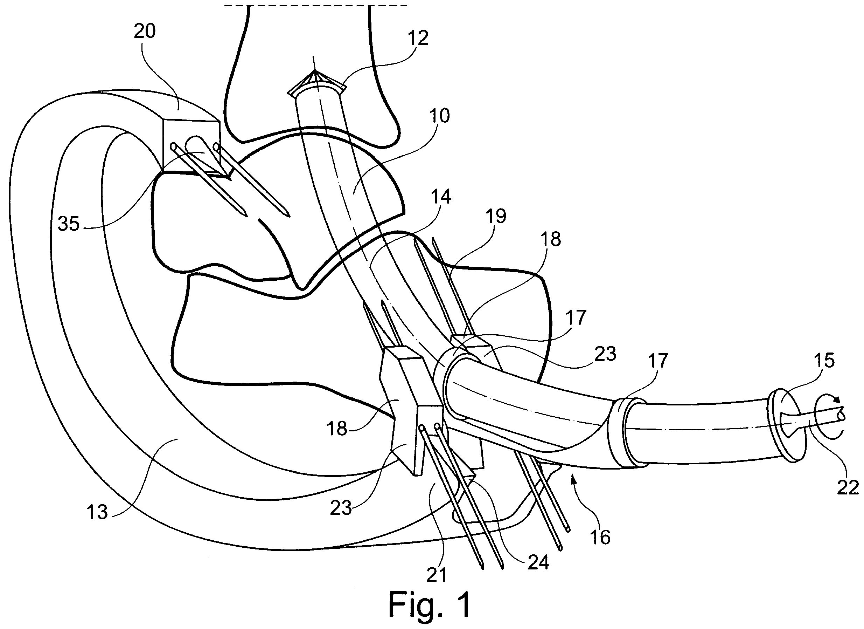

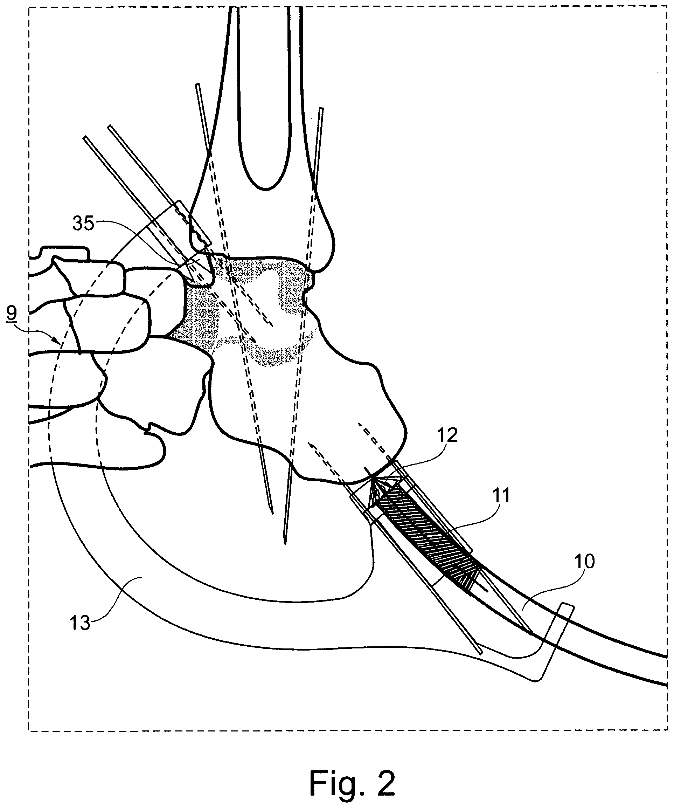

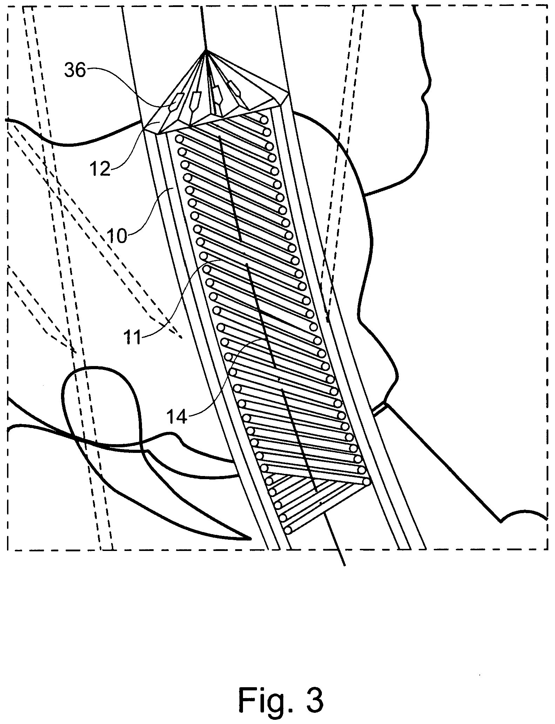

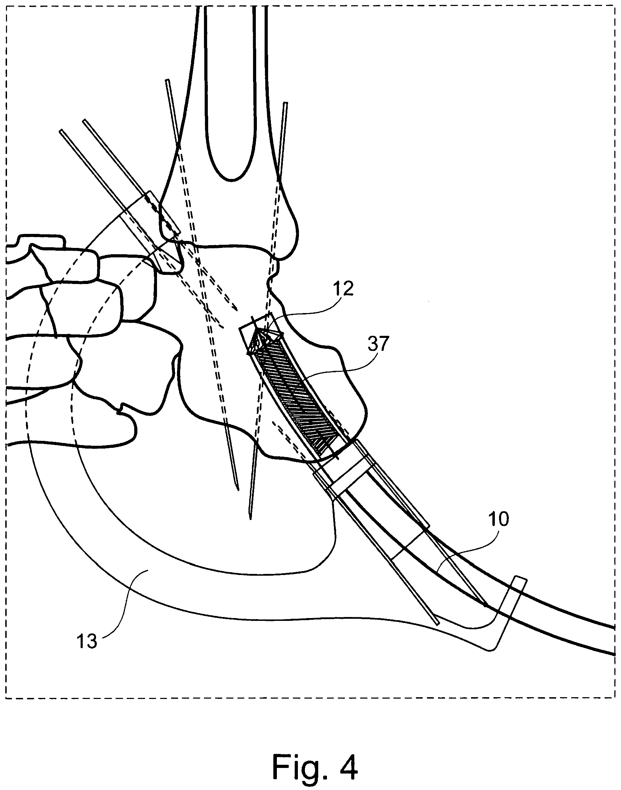

Die in den Fig. 1 bis 4 dargestellte Ausführungsform der Vorrichtung 9 umfasst im wesentlichen ein steifes kreisbogenförmiges Rohr 10 mit einer Zentralachse 14, eine flexible Welle 11 im Inneren des Rohres 10 und einen am vorderen Ende der flexiblen Welle 11 befestigbaren Werkzeugkopf 12. Ferner ist die Vorrichtung 9 mit einem motorischen Antrieb (nicht gezeichnet) für den rotativen Antrieb der flexiblen Welle 11 und des Werkzeugkopfes 12 ausgestattet.

Für den axialen Vorschub ist am hinteren Ende der flexiblen Welle 11 eine Schubplatte 15 fixiert. An der Schubplatte 15 ist endständig ein Antriebszapfen 22 angeordnet, welcher in eine Antriebsmaschine einspannbar ist. Durch Ausüben einer Vorschubskraft auf die Antriebsmaschine kann somit das Rohr 10 zusammen mit der flexiblen Welle 11 vorwärts bewegt werden. Am vorderen Ende der flexiblen Welle 11 sind Kupplungsmittel (nicht gezeichnet) angebracht sind, mittels welcher der Werkzeugkopf 12 an der flexiblen Welle 11 lösbar ankuppelbar ist. Die Kupplungsmittel ermöglichen eine Zugvorspannung der flexiblen Welle 11 nach der Fixation des Werkzeugkopfs 12 am vorderen Ende der flexiblen Welle 11. Die flexible Welle 11 ist mittels der Schubplatte 15 axial fest am hinteren Ende des Rohres 10 gelagert während der Werkzeugkopf 12 axial am vorderen Ende des Rohres 10 anliegt, so dass nach dem Fixieren des Werkzeugkopfes 12 eine Zugvorspannung auf die flexiblen Welle 11 ausgeübt werden kann. Zwischen dem vorderen Ende der flexiblen Welle 11 und dem Werkzeugkopf 12 und zwischen dem hinteren Ende der flexiblen Welle 11 und der Schubplatte 15 ergibt sich eine von der Vorspannung abhängige Reibungskraft. Der Werkzeugkopf 12 wird durch die flexible Welle 11 rotativ und durch das Rohr 10 axial bewegt.
Die Zentralachse 14 des steifen Rohr 10 weist auf der gesamten Länge des Rohres 10 für jeden infinitesimalen Abschnitt einen konstanten Krümmungsradius auf.

Der Werkzeugkopf 12 hat einen Aussendurchmesser D, welcher grösser als der Aussendurchmesser des Rohres 10 ist. Die flexible Welle 11 ist als elastisch biegbare Hohlwelle ausgebildet und weist einen dem Innendurchmesser $d_i$ des Rohres 10 entsprechenden Aussendurchmesser auf. Ferner ist der Werkzeugkopf 12 mit axialen Perforationen 36 zur Abfuhr von Knochenspänen ausgestattet. Die Perforationen 36 durchdringen den Werkzeugkopf 12 in axialer Richtung und münden in den Hohlraum der flexiblen Welle 11. Die während des Bohrvorgangs entstehenden Knochenspäne werden durch die Perforationen 36 in den Hohlraum der flexiblen Welle 11 gefördert und durch diesen abgesaugt.

Die Vorrichtung 9 ist an einer am zu behandelnden Fuss befestigbaren Zielvorrichtung 13 derart bewegbar montiert, dass das steife Rohr 10 zusammen mit der flexiblen Welle 11 und dem Werkzeugkopf 12 axial in den Knochen einschiebbar ist. Die Zielvorrichtung 13 ist als C-Bogen ausgebildet und umfasst eine kreisbogenförmige Führung 16 zur koaxial beweglichen Aufnahme des steifen Rohres 10. In der hier dargestellten Ausführungsform umfasst die kreisbogenförmige Führung 16 zwei Zielringe 17, deren Zentren auf einem Kreisbogen liegen, dessen Radius dem Krümmungsradius der Zentralachse 14 des Rohres 10 entspricht und welcher einen Bogenwinkel von 25° aufweist. Bei einer weiteren (nicht gezeichneten Ausführungsform) sind statt zwei, drei Führungen vorgesehen.

Die Zielvorrichtung 13 umfasst ferner ein an der Oberfläche eines Knochens anlegbares erstes Ende 20 mit einer Spitze 35, ein gabelartig ausgebildetes zweites Ende 21 und einen an der Oberfläche eines Knochens anlegbaren Fixationsschlitten 18. Der Fixationsschlitten 18 umfasst in der hier dargestellten Ausführungsform zwei Backen 23, welche seitlich neben dem steifen Rohr 10 angeordnet sind. Der Fixationsschlitten 18 ist am zweiten Ende 21 der Zielvorrichtung 13 in radialer Richtung bezüglich der bogenförmigen Zielvorrichtung 13 verschiebbar gelagert, wobei die zwei Backen 23 mittels in radialer Richtung verlaufenden Schwalbenschwanzführungen 24 geführt sind. Die zwei Backen 23 sind mit zur Ebene der Zentralachse 14 des steifen Rohres 10 parallel angeordneten Bohrungen zur Aufnahme von Kirschnerdrähten 19 versehen, so dass die Zielvorrichtung 13 mittels Kirschnerdrähten 19 am Knochen befestigbar ist. Die Montage des Werkzeugkopfes 12 erfolgt nach dem Fixieren der Zielvorrichtung 13 am Fuss, wobei das steife Rohr 10 so weit zurückgezogen wird, dass der Werkzeugkopf 12 zwischen dem vorderen Ende des steifen Rohres 10 und der Knochenoberfläche und zwischen den zwei Backen 23 des Fixationsschlittens 18 eingeführt und am vorderen Ende der flexiblen Welle 11 befestigt werden kann.

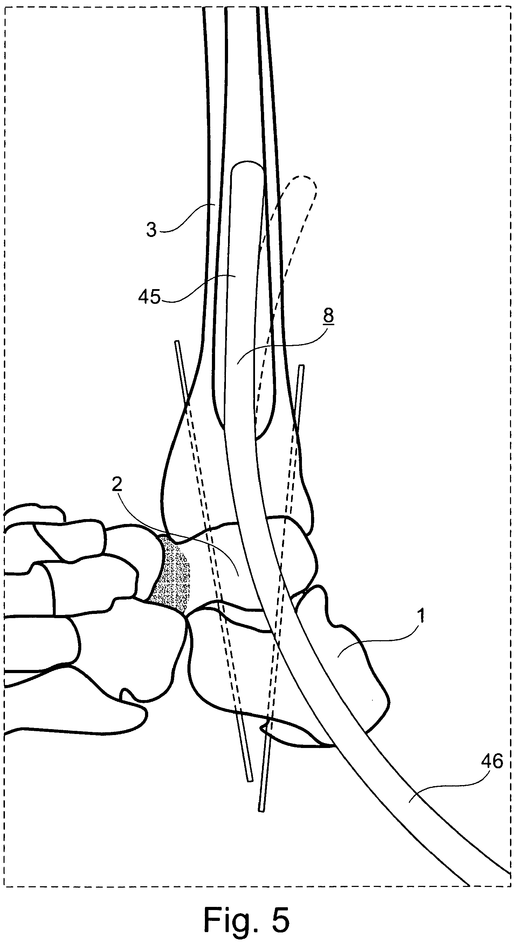

Die Fig. 5 zeigt den Unterschenkel in einer Position, in welcher er um ca. 45° nach Innen gedreht ist. Es sind diejenigen Knochen dargestellt, welche für die Anwendung des erfindungsgemässen Implantats 8 von Bedeutung sind. Es handelt sich primär um den Calcaneus 1, den Talus 2 und die Tibia 3. Ebenfalls in Fig. 5 eingezeichnet ist der erfindungsgemäss als Implantat 8 ausgebildete Knochennagel, welcher durch den Calcaneus 1 und den Talus 2 und teilweise bis in den unteren Teil der Tibia 3 eingeführt ist. Vor der Implantation hat der vordere Teil 45 des Implantats 8 eine gekrümmte Form (gestrichelt dargestellt) mit demselben Krümmungsradius R wie der hintere Teil 46. Durch die geringere Steifigkeit des vorderen Teils 45 kann sich dieser während des Einbringens in die Tibia 3 deformieren, so dass der vordere Teil 45 nach der Implantation einen erheblich grösseren Krümmungsradius aufweisen kann oder sogar geradlinig geformt sein kann.

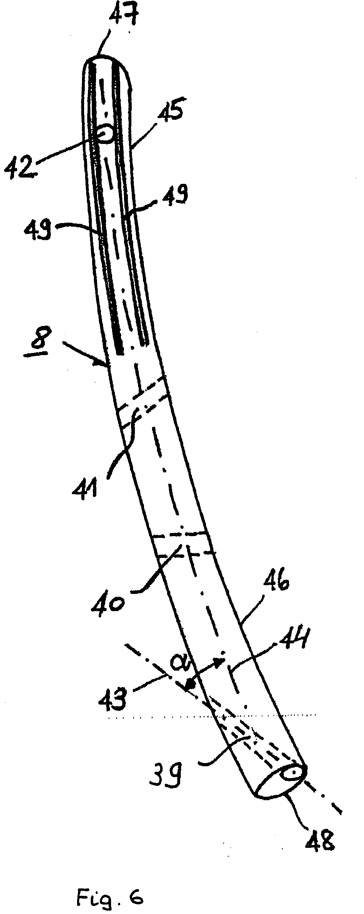

Wie in Fig. 6 dargestellt ist das Implantat 8 in einer Ebene stetig gekrümmt mit einem Krümmungsradius R von typischerweise 190 mm. Die Krümmung erstreckt sich über die gesamte Länge $L_I$ des Implantats 8, welche typischerweise 140 mm beträgt. Sein Durchmesser $D_I$ beträgt typischerweise 11 mm. Das Verhältnis $L_I/D_I$ zwischen Länge $L_I$ und Durchmesser $D_I$ des Implantats 8 beträgt typischerweise 12,7. Das Implantat 8 weist eine Anzahl von Querbohrungen 39, 40, 41, 42 auf, in welche Verriegelungsschrauben 30,31,32 eingeführt werden können. Drei der Querbohrungen 39, 41, 42 liegen in der gleichen Ebene in welcher das Implantat 8 gekrümmt ist. Eine vierte Querbohrung 42 liegt am proximalen Ende des Implantats 8 und verläuft senkrecht zur erwähnten Krümmungsebene. Die Längsachse 43 der am distalen Ende des Implantats 8 liegenden Querbohrung 39 schliesst mit der Längsachse 44 des Implantats 8 einen Winkel alpha von 15° ein.

Das Implantat 8 besteht aus einem insbesondere biegesteifen Material. Am vorderen Teil 45 des Implantates 8 sind Längsschlitze 49 angebracht, so dass der vordere Teil 45 des Implantats 8 elastisch biegbar ausgebildet ist. Um das Implantat 8 in den Knochen einführen zu können, ist es je nach Knochenqualität vorteilhaft die betroffen Knochen 1, 2, 3 zuvor aufzubohren. Dies geschieht mittels der in den Fig. 1 bis 4 dargestellten Vorrichtung 9.

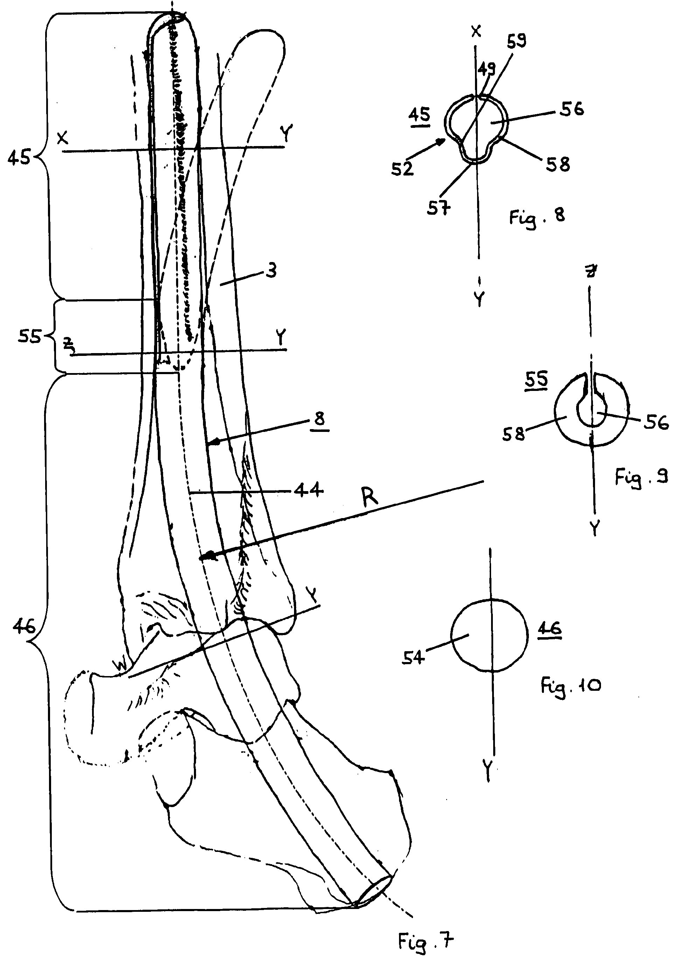

In den Fig. 7 - 10 ist eine weitere Ausführungsform des Implantates 8 in Form eines Knochennagels dargestellt, welche sich von der Ausführungsform gemäss Fig. 6 nur darin unterscheidet, dass der Knochennagel:

a) in seinem vorderen Teil 45 ein kleeblattförmiges Querschnittsprofil 52 (Fig. 8), einen zur Längsachse 44 parallelen Hohlraum 56 und einen zur Längsachse 44 parallelen Längsschlitz 49 umfasst; und

b) zwischen seinem vorderen Teil 45 und seinem hinteren Teil 46 einen intermediären Teil 55 umfasst, welcher als Übergangsbereich zwischen dem vorderen Teil 45 mit kleiner Steifigkeit und dem hinteren Teil 46 mit hoher Steifigkeit ausgebildet ist, so dass die Steifigkeit des Knochennagels gegen den hinteren Teil 46 kontinuierlich zunimmt.

Durch den Hohlraum 56 erhält der Knochennagel im vorderen Teil 45 ein dünnwandiges Querschnittsprofil 52 mit einer peripheren eine geringe Wandstärke aufweisenden Wand 58, welche zudem durch den Längsschlitz 49 parallel zur Längsachse 44 durchtrennt ist. Die Kleeblattform im Querschnittsprofil 52 ergibt sich durch die Ausbildung der Wand 58 mit zwei sich parallel zur Längsachse 44 erstreckenden Einbuchtungen 59 im vorderen Teil 45. Die zwei Einbuchtungen 59 sind derart auf dem Umfang des Knochennagels angeordnet, dass eine dem Längsschlitz 49 diametral gegenüberliegende, sich ebenfalls parallel zur Längsachse erstreckende konvexe Ausbuchtung 57 der Wand 58 gebildet wird. Im intermediären Teil 55 nimmt die Steifigkeit vom vorderen Teil 45 gegen den hinteren Teil 46 durch eine gegen den hinteren Teil 46 zunehmende Wandstärke der Wand 58 zu. Diese zunehmende Wandstärke wird einerseits durch ein kontinuierliches Verengen des Hohlraums 56 und andererseits dadurch erreicht, dass die radiale gemessene Tiefe der Einbuchtungen 59 in der Wand 58 gegen den hinteren Teil 46 kontinuierlich abnimmt, so dass ein kontinuierlicher Übergang vom kleeblattförmigen Querschnittsprofil 52 im vorderen Teil 45 zum kreisförmigen Querschnittsprofil 54 im hinteren Teil 46 gebildet wird. Vor der Implantation hat der intermediäre Teil 55 und der vordere Teil 45 des Knochennagels eine gekrümmte Form (gestrichelt dargestellt) mit demselben Krümmungsradius R wie der hintere Teil 46. Durch die geringere Steifigkeit des intermediären Teils 55 und insbesondere des vorderen Teils 45 können sich diese während des Einbringens in die Tibia 3 deformieren, so dass der vordere Teil 45 nach der Implantation einen erheblich grösseren Krümmungsradius aufweisen kann oder sogar geradlinig geformt sein kann (Fig. 7).

Beschreibung der Operationstechnik:

1) Patient in Rücken-, Bauch-, oder Seitenlage bringen;

2) durch einen gezielten, begrenzten, vorderen, Seiten-, oder postero-lateralen Zugang werden beide Gelenke (oberes und subtalares) angegangen und der Restknorpel entfernt;

3) Tibia 3, Talus 2 und Calcaneus 1 sollen frei beweglich sein um die gewünschte Reorientierung (Einstellung) zu erreichen;

4) Einstellung der zerstörten und zu arthrodesierenden Gelenke zwischen Calcaneus, Talus und Tibia;

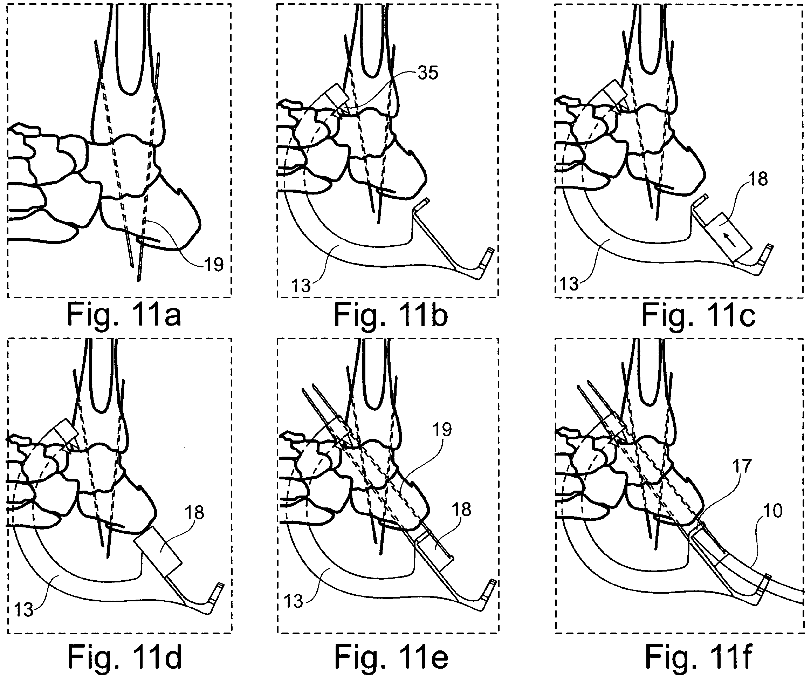

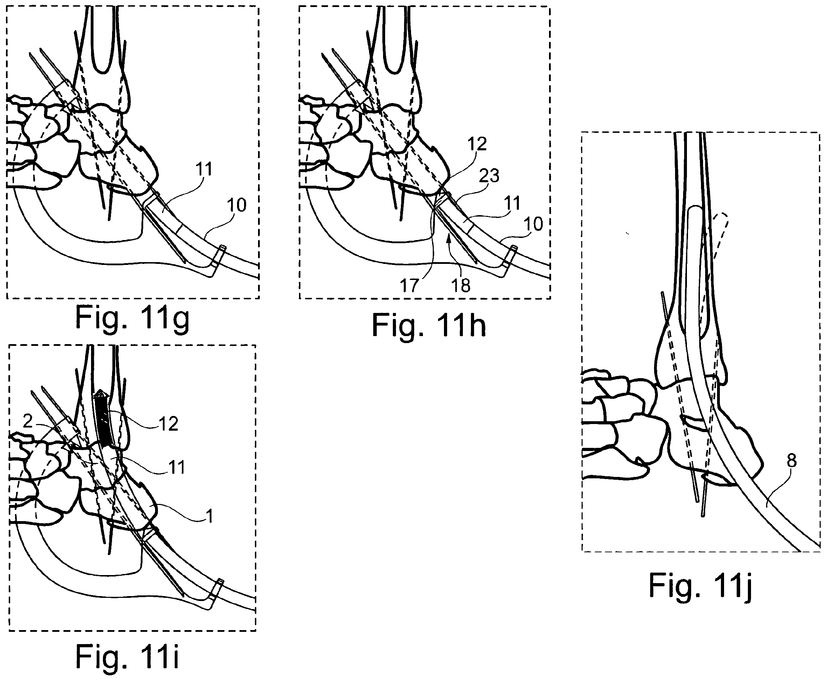

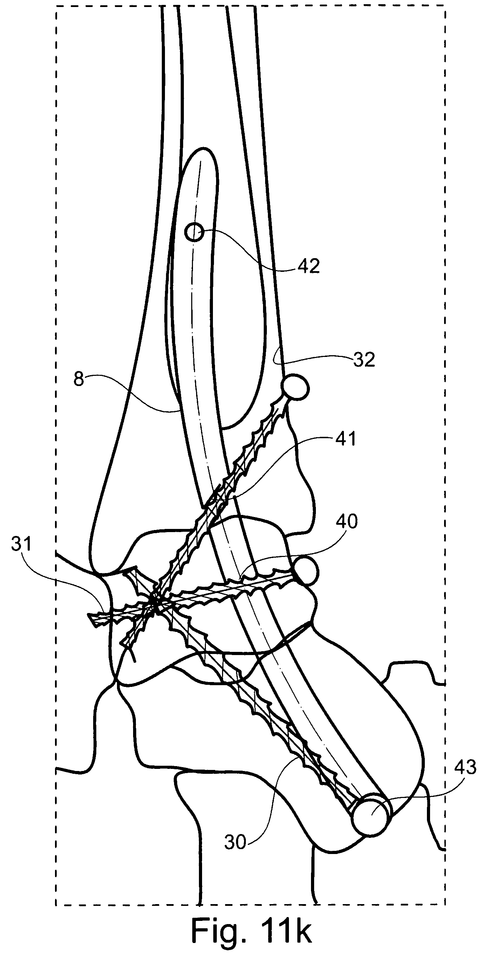

5) Die angezielte Position wird mit perkutan eingebrachten, festen Kirschnerdrähten 19 (3 mm Durchmesser) fixiert (Fig. 11 a);

6) Zugang von ca. 2 - 3 cm Länge an der lateralen Ferse herstellen;

7) Aufsuchen des postero-lateralen Calcaneusrandes (tuber calcanei);

8) Anlegen der Zielvorrichtung 13 mit der Spitze 35 der Zielvorrichtung 13 am medialen Ansatz des oberen Sprunggelenks (Fig. 11 b);

9) Montage des Fixationsschlittens 18 an der Zielvorrichtung 13 (Fig. 11 c);

10) Verschieben des Fixationsschlittens 18 in Hautnähe beim Zugang (Fig. 11 d);

11) Fixation der Zielvorrichtung 13 mit dem Fixationsschlitten 18 an der Ferse, ebenfalls mit Kirschnerdrähten 19 (Fig. 11 e);

12) Einführen des steifen Rohres 10 in die zwei Zielringe 17 (Fig. 11f);

13) Einführen der flexiblen Welle 11 in das steife Rohr 10 (Fig. 11g);

14) Befestigen des Werkzeugkopfes 12 am vorderen Ende der flexiblen Welle 11 und Einbringen in die Knochenöffnung (Fig. 11 h). Der Werkzeugkopf 12 wird zwischen der Fussoberfläche und dem vorderen Zielring 17 und zwischen den zwei Backen 23 des Fixationsschlittens 18 eingeführt und unter Vorspannung der flexiblen Welle 11 an dieser befestigt. Dazu werden unter den Schritten 12 und 13 das Rohr 10 und die flexible Welle 11 nur soweit in die zwei Zielringe 17 vorgeschoben, bis die vorderen Enden des Rohres 10 und der flexiblen Welle 11 mit der Stirnfläche des vorderen Zielrings 17 übereinstimmen;

15) Fräsen der Bohrung für das Implantat 8 durch simultanes Rotieren und Schieben der flexiblen Welle 11 mit einer Durchquerung des Calcaneus 1 und des Talus 2 auf Höhe der posterioren subtalaren Gelenksfazette bis der Werkzeugkopf 12 in den Tibamarkraum gelangt (Fig. 11 i);

16) Demontieren der Vorrichtung 9;

17) Einführen des Implantates 8 in die Bohrung bis in die Tibia - Markhöhle (Fig. 11j) mittels Einbringen des Implantates 8 mit eventuellem Zielgriff (nicht gezeichnet), welcher Führungen zum Bohren der Bohrungen für die Verrieglungsschrauben 30,31,32 enthält;

15) Einschlagen des Nagels 33, bis sich dessen vorderer Teil 45 in der Tibia-Markhöhle plastisch streckt (Fig. 5);

16) Einbringen perkutan der eventuellen Verrieglungsschrauben 30,31,32 für den Calcaneus 1, den Talus 2, und die Tibia 3 (Fig. 11 k); und

17) Anbringen der Hautnaht.

In Fig. 11 k ist der Fuss mit dem Unterschenkel um ca. 45° nach Innen gedreht. Das Implantat 8 ist in seiner endgültigen implantierten Lage dargestellt. Eine erste Verriegelungsschraube 30 in der Querbohrung 39 sichert das Implantat 8 im Calcaneus 1 und im Talus 2. Eine zweite Verrieglungsschraube 31 in der Querbohrung 40 blockiert das Implantat 8 im Talus 2. Eine dritte Verrieglungsschraube 32 in der Querbohrung 41 blockiert das Implantat 8 in der Tibia 3 und im Talus 2. Somit werden die drei Knochen Tibia 3, Talus 2 und Calcaneus 1 zusammen versteift und zwar - was wichtig ist - in einer anatomisch richtigen Stellung.

**Patentansprüche**

1. Vorrichtung (9) zum Bohren einer kreisbogenförmigen Knochenbohrung, umfassend:

    - ein steifes kreisbogenförmiges Rohr (10) mit einer Zentralachse (14);
    - eine im Inneren des Rohres (10) einführbare oder eingeführte flexible Welle (11);
    - einen am vorderen Ende der flexiblen Welle (11) befestigbaren Werkzeugkopf (12).

    **dadurch gekennzeichnet, dass**

die Krümmung für jeden infinitesimalen Abschnitt des steifen Rohres (10) einen Krümmungsradius der Zentralachse (14) aufweist, der mindestens 130 mm beträgt.

2. Vorrichtung (9) nach Anspruch 1, **dadurch gekennzeichnet, dass** der Werkzeugkopf (12) einen Aussendurchmesser D aufweist, und dass das Verhältnis $d_a/D$ zwischen dem Rohraussendurchmesser $d_a$ und dem Aussendurchmesser D maximal 0,95 beträgt.

3. Vorrichtung (9) nach Anspruch 2, **dadurch gekennzeichnet, dass** das Verhältnis $d_a/D$ zwischen dem Rohraussendurchmesser $d_a$ und dem Aussendurchmesser D maximal 0,92 beträgt.

4. Vorrichtung (9) nach einem der Ansprüche 1 bis 3 zusammen mit einer am zu behandelnden Körperteil befestigbaren Zielvorrichtung (13), welche eine kreisbogenförmige Führung (16) aufweist zur beweglichen Aufnahme des steifen Rohres (10).

5. Vorrichtung (9) nach Anspruch 4, **dadurch gekennzeichnet, dass** die Führung (16) zwei oder mehr Zielringe (17), vorzugsweise drei Zielringe (17) umfasst.

6. Vorrichtung (9) nach einem der Ansprüche 1 bis 5, **dadurch gekennzeichnet, dass** der Werkzeugkopf (12) mit axialen Perforationen (36) zur Abfuhr von Knochenspänen ausgestattet ist.

7. Kit umfassend eine Vorrichtung (9) nach einem der Ansprüche 1 bis 6 und mindestens ein als Knochennagel ausgebildetes Implantat (8) für die Ferse mit:

    A) einem vorderen zur Einführung in den Knochen bestimmten Teil (45) mit einer Spitze (47);
    B) einem hinteren Teil (46) mit einem Ende (48), wobei
    C) der Knochennagel in einer Ebene gekrümmt ist; und
    D) die Krümmung für jeden infinitesimalen Abschnitt des Knochennagels einen Krümmungsradius aufweist, der mindestens 130 mm beträgt.

8. Kit nach Anspruch 7, **dadurch gekennzeichnet, dass** der hintere Teil (46) auf einer Länge von vorzugsweise mindestens 120 mm, vom Ende (48) aus gerechnet, steif ist.

9. Vorrichtung (9) nach einem der Ansprüche 1 bis 6 zur Verwendung für die tibio-calcaneare Arthrodese.

## Claims

1. A device (9) for drilling a circular arc-shaped bone borehole, comprising:

   - a stiff circular arc-shaped tube (10) with a central axis (14);
   - a flexible shaft (11) that is insertable or inserted into the interior of the tube (10);
   - a tool head (12) that is attachable to the front end of the flexible shaft (11),

   **characterized in that,**
   the curvature for each infinitesimal segment of the stiff tube (10) has a radius of curvature of the central axis (14) of at least 130 mm.

2. The device (9) as set forth In claim 1, **characterized in that** the tool head (12) has an outer diameter D and that the ratio $d_a/D$ between the tube outer diameter $d_a$ and the outer diameter D is no greater than 0.95.

3. The device (9) as set forth in claim 2, **characterized in that** the ratio $d_a/D$ between the tube outer diameter $d_a$ and the outer diameter D Is no greater than 0.92.

4. The device (9) as set forth in any one of claims 1 to 3 together with a target device (13) that is attachable to the body part to be treated and has a circular arc-shaped guide (16) for movably receiving the stiff tube (10).

5. The device (9) as set forth in claim 4, **characterized in that** the guide (16) comprises two or more target rings (17), preferably three target rings (17).

6. The device (9) as set forth in any one of claims 1 to 5, **characterized in that** the tool head (12) is outfitted with axial perforations (36) for discharging bone chips.

7. A kit comprising a device (9) as set forth in any one of claims 1 to 6 and at least one implant (8) for the heel embodied as a bone nail, with:

   A) a front part (45) intended for insertion into the bone with a tip (47);
   B) a rear part (46) with an end (48),
   C) the bone nail being curved on a plane, and
   D) the curvature having a radius of curvature for each infinitesimal segment of the bone nail that is at least 130 mm.

8. The kit as set forth in claim 7, **characterized in that** the rear part (46) is stiff on a length of preferably at least 120 mm as calculated from the end (48).

9. The device (9) as set forth in any one of claims 1 to 6 for use for tibiocalcaneal arthrodesis.

## Revendications

1. Dispositif (9) pour percer un trou en arc de cercle dans un os, comprenant :

   - un tube (10) rigide en forme d'arc de cercle avec un axe central (14) ;
   - un arbre (11) flexible pouvant être introduit ou étant introduit dans l'intérieur du tube (10) ;
   - une tête d'outil (12) pouvant être fixée sur l'extrémité avant de l'arbre (11) flexible,

   **caractérisé en ce que**

   la courbure présente, pour chaque segment Infinitésimal du tube (10) rigide, un rayon de courbure de l'axe central (14) qui est au moins égal à 130 mm.

2. Dispositif (9) selon la revendication 1, **caractérisé en ce que** la tête d'outil (12) présente un diamètre extérieur D, et **en ce que** le rapport $d_a/D$ entre le diamètre extérieur de tube $d_a$ et le diamètre extérieur D est au maximum égal à 0,95.

3. Dispositif (9) selon la revendication 2, **caractérisé en ce que** le rapport $d_a/D$ entre le diamètre extérieur de tube $d_a$ et le diamètre extérieur D est au maximum égal à 0,92.

4. Dispositif (9) selon l'une des revendications 1 à 3, conjointement avec un dispositif de visée (13), pouvant être fixé sur la partie du corps à traiter, qui présente un guidage (16) en forme d'arc de cercle pour la réception mobile du tube (10) rigide.

5. Dispositif (9) selon la revendication 4, **caractérisé en ce que** le guidage (16) comprend deux anneau de visée (17) ou davantage, de préférence trois anneaux de visée (17).

6. Dispositif (9) selon l'une des revendications 1 à 5, **caractérisé en ce que** la tête d'outil (12) est équipée de perforations (36) axiales destinées à l'évacuation des copeaux d'os.

7. Kit comprenant un dispositif (9) selon l'une des revendications 1 à 6 et au moins implant (8) pour le talon constitué en tant que clou à os, avec :

   A) une partie (45) avant, avec une pointe (47), destinée à être introduite dans l'os ;
   B) une partie (46) arrière avec une extrémité (48), dans lequel

C) le clou à os est courbe dans un plan ; et

D) la courbure, pour chaque segment infinitésimal du clou à os, présente un rayon de courbure qui est au moins égal à 130 mm.

8. Kit selon la revendication 7, **caractérisé en ce que** la partie (46) arrière est rigide sur une longueur de préférence d'au moins 120 mm en comptant à partir de l'extrémité (48).

9. Dispositif (9) selon l'une des revendications 1 à 6, destiné à être utilisé pour l'arthrodèse tibio-calcanéenne.

Fig. 1

Fig. 2

Fig. 3

Fig. 4

Fig. 5

Fig. 6

Fig. 7

Fig. 8

Fig. 9

Fig. 10

Fig. 11c

Fig. 11b

Fig. 11a

Fig. 11f

Fig. 11e

Fig. 11d

Fig. 11j

Fig. 11h

Fig. 11g

Fig. 11i

Fig. 11k

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2008099176 A1
- US 20080140078 A1
- US 20070225721 A1
- US 1630239 A
- DE 7607981

### In der Beschreibung aufgeführte Nicht-Patentliteratur

- **DUBBEL.** Taschenbuch für den Maschinenbau. Springer-Verlag, 2005